# EUROPEAN PATENT SPECIFICATION

(11) **EP 0 483 070 B1**
(45) Date of publication and mention of the grant of the patent: **12.04.1995**
(21) Application number: 91810813.5
(22) Date of filing: 22.10.1991
(51) Int. Cl.: A23L 1/308, A23L 1/052, A23L 1/054, A23L 1/056

(54) **Nutritionally complete feeding composition containing hydrolysed soluble fiber**
Vollwertnahrung aus hydrolysierten löslichen Faserstoffen
Aliment complet contenant des fibres solubles hydrolysées

(30) Priority: 24.10.1990 US 602531
(43) Date of publication of application: 29.04.1992
(73) Proprietor: Novartis Nutrition AG, 3007 Bern (CH)
(72) Inventor: Greenberg, Norman A., New Hope, Minnesota 55427 (US)
(74) Representative: Smolders, Walter

(56) References cited:
- EP-A- 0 385 598
- GB-A- 2 201 875
- PATENT ABSTRACTS OF JAPAN vol. 15, no. 385, 27 September 1991; & JP - A - 3155768 (NICHIRO CORP) 03.07.1991
- BAKERS DIGEST vol. 58, no. 4, July/August1984, pages 32,33, Chicago, Illinois, US; "Soluble dietary fiber gives marketing and nutritional benefits"

## Description

This application relates to low-viscosity enteral and medical foods which contain hydrolysed soluble fiber, and to the use of these foods to maintain healthy colon cells and to prevent bacterial sepsis.

Nutritionally complete liquid diets are often administered to patients either orally or through feeding tubes (enterally). It has been noted that a frequent side-effect of this type of feeding is diarrhea. Diarrhea can lead to fluid and/or electrolyte imbalance and malnutrition. Further, it can cause discomfort and sanitation problems, require considerable staff time, thus resulting in increase patient care costs.

It has also been noted that many critically ill patients develop bacterial sepsis, a leading cause of death in intensive care units.

Feeding compositions are currently known which contain soy polysaccharide fiber. Soy polysaccharide fiber is considered to be an insoluble fiber. A recent study questioned the effectiveness of such a formulation in preventing diarrhea. See, e.g. Frankenfield et al., 1989. "Soy-Polysaccharide Fiber: Effect on Diarrhea in Tube-fed, Head Injured Patients" Am. J. Clin. Nutr. 50:533-538. Whilst certain soluble fibers, in particular pectin, have been proposed for treatment or prevention of diarrhea, these products have not been successfully applied in enteral formulation for technological reasons, i.e. the occurrence of extreme product thickening after the required heat treatment of enteral formulations comprising soluble fibres.

### Description of the Invention

It has been found that adding a hydrolysed soluble fiber supplement to an otherwise nutritionally complete liquid food composition can control diarrhea and prevent bacterial sepsis and gut atrophy while retaining the low viscosity character of the food composition. This invention, therefore relates to food compositions comprising such hydrolysed soluble fiber, and to the use of such food compositions to prevent diarrhea, bacterial sepsis and gut atrophy.

As used throughout the specification and claims, the term "nutritionally complete" refers to a feeding composition which contains carbohydrates, proteins, essential fatty acids, vitamins, and minerals in such amounts that a person can ingest only that composition for a prolonged period of time and not suffer any malnutrition. The composition may have water added to it such that the composition is in liquid form and suitable for drinking or for use with a tube-feeding apparatus. Alternatively, the composition may be in dry form.

Numerous feeding compositions are known and commercially available, including those commercially available from Sandoz Nutrition Corp. under the trademarks RESOURCE^{R} and ISOSOURCE^{R} (both liquid formulations), STRESSTEIN^{R} (dry product), NUTRODRIP^{R} and IMPACT^{R}. These compositions typically provide approximately 20-70 % of calories in the form of carbohydrates, 13-30 % of calories in the form of protein, 20-50 % of calories as lipid (which includes essential fatty acids) as well as vitamins, minerals, and optionally water, flavoring agents, fillers, binders, coloring agents, coating materials, or other nutritional supplements.

As used throughout the specification and claims, the term "soluble fiber" refers to fibers which are able to undergo fermentation in the colon to produce short chain fatty acids (SCFA). Examples of soluble fibres are: pectin, guar, and gum arabic.

It has been found in accordance with this invention that hydrolysed soluble fibers can be added to feeding compositions, and result in a composition which prevents diarrhea, bacterial sepsis and gut atrophy. The hydrolysed soluble fiber may be the only fiber in the feeding composition, i.e. it may be added to a feeding composition which previously did not contain any fiber, or it may replace fiber which previously was present in the feeding composition. Alternatively, the hydrolysed soluble fiber may be an addition to a non-hydrolysed soluble fiber and/or an insoluble fiber such as soy polysaccharide present in the feeding composition.

The hydrolysed soluble fiber may be derived from numerous known soluble fibers, including from locust bean gum, xanthan gum, guar gum, and pectin. The preferred fibers, for numerous reasons set forth below are hydrolysed guar gum and hydrolysed pectin; hydrolysed guar gum being the most preferred. The term hydrolysed soluble fibers as used herein refers to soluble fibers hydrolysed in conventional manner, e.g. chemically or enzymatically to soluble fibers having a reduced molecular weight, which hydrolysed products are tube compatible when administered at the desired daily amount.

One primary requirement for tube compatibility of enteral compositions, is that the hydrolysed soluble fiber should not substantially increase the viscosity of the product above approximately 0,05 Pa·s (50 cp), and it is preferred that the viscosity remains under 0,025 Pa·s (25 cp), more preferably 0,01 Pa·s (10-25 cp). As used throughout the specification and claims, the term "low viscosity" means a viscosity of less than 0,05 Pa·s (50 cp), preferably less of 0,025 Pa·s (25 cp).

For use as enteral formulation (tube feeding) the formulations are essentially neutral in nature, i.e. have a pH of about 7.

A particularly preferred hydrolysed guar gum is commercially available from Taiyo Kagaku Co, Ltd. (Japan) under the trade name SUN FIBER^{R}. SUNFIBER^{R} is a purified hydrolysed guar gum prepared by hydrolysing guar gum with β-mannase from Aspergillus niger comprising ca. 75 % by weight of soluble fiber. Prior to hydrolysis, the molecular weight of guar gum is approximately 200,000; after hydrolysis it is 20,000-30,000. For use in accordance with this invention, the molecular weight range of the hydrolysed guar gum may vary, as long as the viscosity of the finished product does not exceed 0,05 Pa·s (50 cp).

The amount of hydrolysed soluble fiber added to a feeding composition may vary depending on the needs of the patient and whether the composition is to be taken orally or enterally. Thus the fiber content of the composition may vary according to the amount of composition intended to be ingested per day. It is generally preferred that the hydrolysed soluble fiber content of the composition be adjusted so that the patient receives approximately 10-60 g/day soluble fiber, more preferably approximately 10-45 g/day soluble fiber e.g. from 10-30 g/day, particularly from 20-30 g/day soluble fiber.

Such liquid formulations provide conveniently up to 3000 kcal, e.g. 1500 to 2500 kcal per day. They have, in general, a hydrolyzed soluble fiber content which lies in the range of from 0.3 to 10 gram, preferably from 0.5 to 7.5 gram, more preferably of from 0.6 to 3.0 gram soluble fiber per 100 ml formulation.

It is well known that colon cells nourished by exclusively liquid diets may atrophy. This atrophy manifests itself in a breaking down of the gut mucosal barrier, allowing gram negative bacterial and/or bacterial endotoxin produced by these bacteria to invade the patient's circulatory system, causing shock. It has been found in accordance with this invention, that providing colon cells with a source of hydrolysed fiber which can be fermented into butyric acid, maintains health of the colon cells and the intactness of the gut mucosal barrier, reducing the incidence of septic shock. In addition, the healthy colon cells can retain minerals better, and are able to reabsorb water. Hydrolysed pectin and hydrolysed guar gum are particularly good sources of butyric acid, as detailed in Example 1 below.

Another benefit which can be realised by the addition of hydrolysed soluble fiber such as hydrolysed guar gum or hydrolysed pectin is that diarrhea can be controlled (treated or prevented).

This invention is further illustrated in the following non-limiting examples.

### EXAMPLE 1

### Fermentability of Fibers

Various fibers are suspended in water and buffered to neutrality. A sample of micro-organisms normally found in the colon is added and fermentation is allowed to occur for 24 hours at 37°C in a manometer. The resulting ferment is analysed for the amount of butyric acid per gram of fiber. Results are presented below:

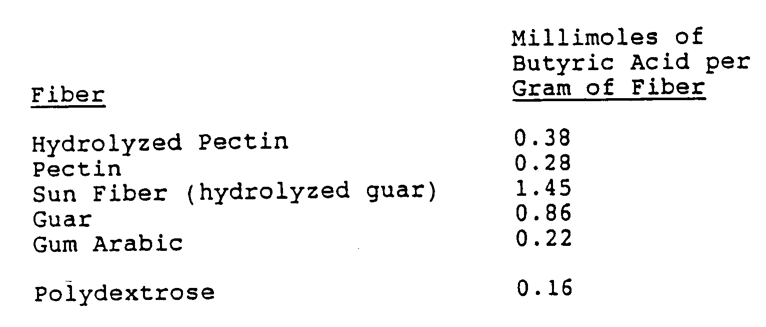

As can be seen from the above, the hydrolysis of both pectin and guar improves the fermentability of these fibers into butyric acid.

### EXAMPLE 2

### Oral Supplement Containing Soluble Fiber

An oral supplement is made using the following ingredients.

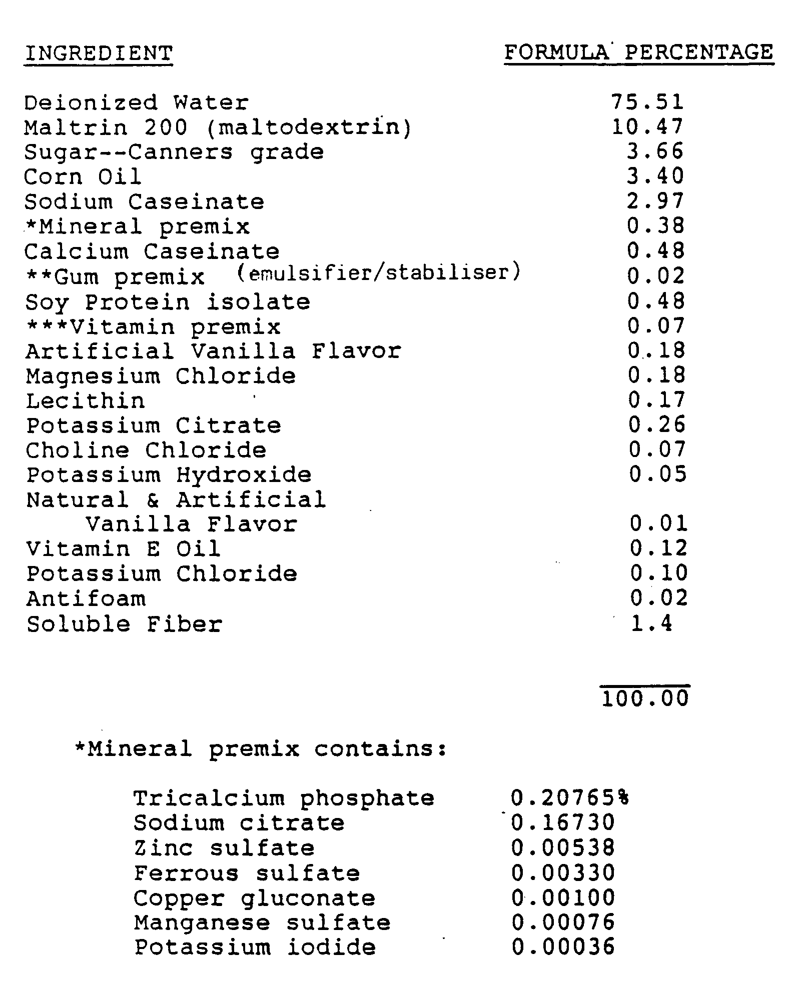

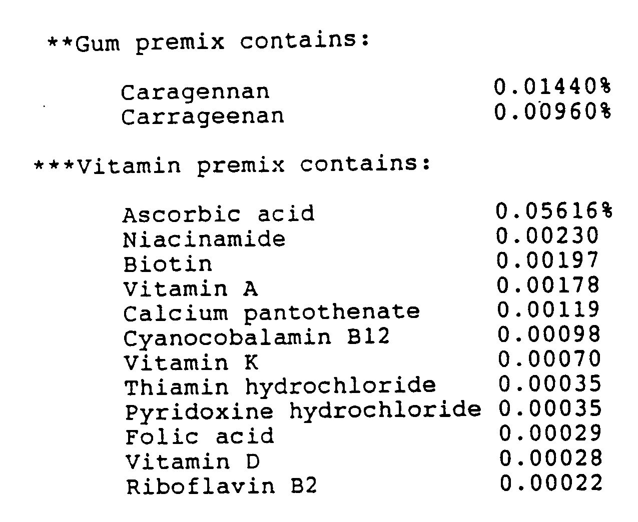

Oil is heated to 68,3-73.9°C (155-165°F). Wet salts and gum premix is added to the deionized water which is heated to 60°C (140°F). Next, the maltrin, sugar, caseinates, and soy protein is added. The soluble fiber source is then added. The soluble fiber may be either hydrolyzed guar gum or pre-treated pectin (at least 0.85%, which equals at least 15 g per 2,000 calories). pH is adjusted to 6.8. Next, the potassium citrate and the mineral premix are added and temperature is increased to 65,6°C (150°F). The hot oil is then added and heated to 73,9°C (165°F). The mixture is then homogenized. Vitamin premix and flavor is added and mixed thoroughly. The mixture is then heat processed and packaged aseptically.

### EXAMPLE 3

### Replacing Insoluble Fiber with Soluble Fiber

A currently available product, FIBERSOURCE®, contains soy polysaccharide which is insoluble fiber. Hydrolyzed guar gum may be used to replace the soy polysaccharide or may be added in addition to the soy polysaccharide. Below is an example where the hydrolyzed guar gum replaces soy polysaccharide on a one-to-one basis, but other ratios may be used. If one wished to replace soy polysaccharide with hydrolyzed pectin, a different emulsifier system is needed.

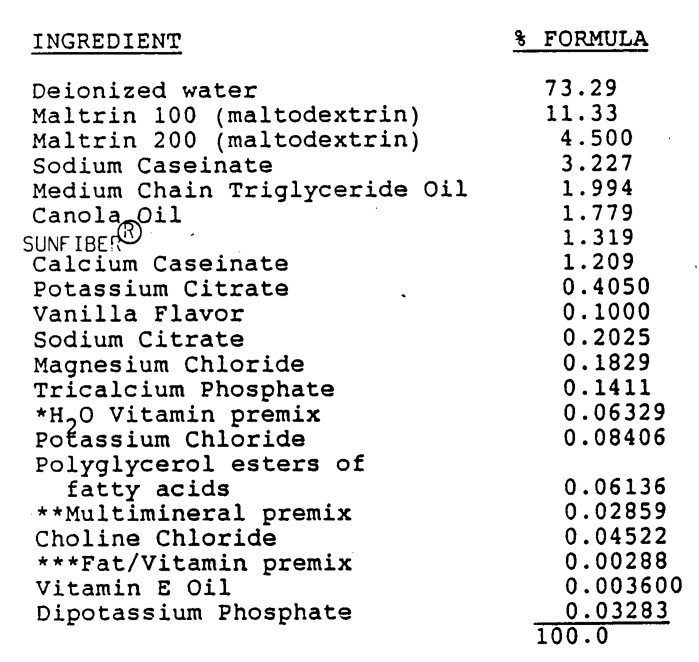

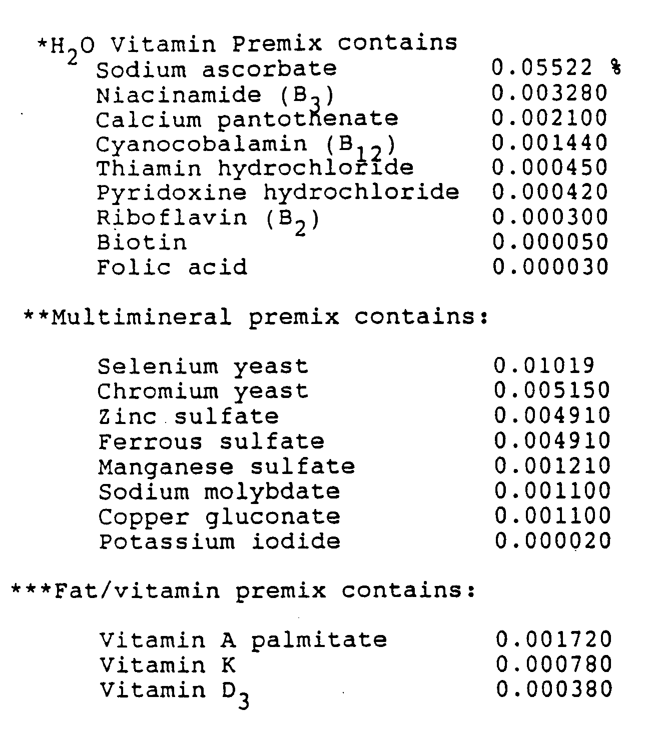

### EXAMPLE 4

Effects of Dietary Soluble and Insoluble Fiber on the Intestinal Flora, Intestinal Histology and Bacterial Transaction in Mice.

### 1. Effect on Bacteria

Four groups of mice are fed either normal mouse chow, a commercially available liquid food composition (abbreviated Liq), the liquid composition supplemented with 2.5 % soy fiber (Liq+S), or the liquid composition supplemented with 2.5 % SUNFIBER^{R} (Liq+G). After 14 days, the type and amount of cecal bacteria are measured. Results are presented in Table 4A below. "Wt" is average (n=24) weight gain in grams in 14 days. Numbers given under the bacterial columns represent the average and standard error (log₁₀) of cecal bacteria per gram (n=8)

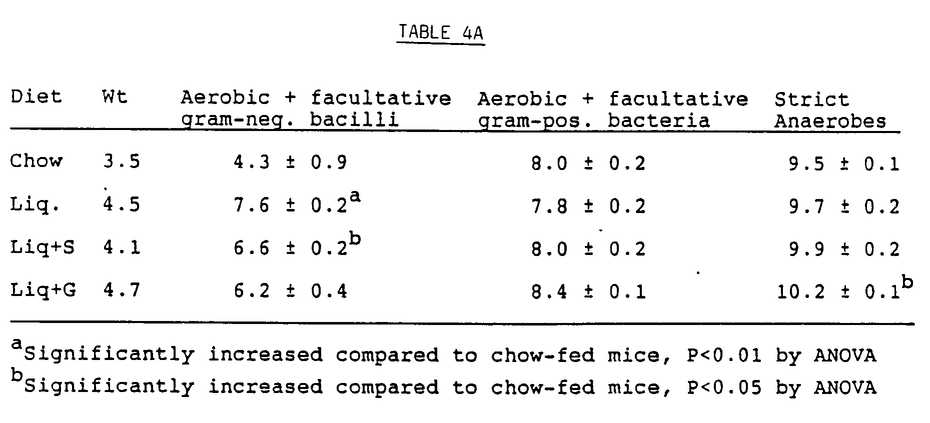

The table shows that the liquid diet and the liquid diet supplemented with soy fiber both significantly increased the amount of aerobic and facultative gram-negative bacteria. Gram-negative bacteria are those which can cause bacterial sepsis if they or the endotoxins produced by them translocate into the bloodstream. No statistically significant increase was observed in the liquid food supplemented with the hydrolysed guar. The strict anaerobe count was increased with the liquid + hydrolysed guar but these bacteria are considered more "benign" than the aerobic gram-negative bacteria.

### 2. Translocation of bacteria

Septic shock occurs after bacteria or their endotoxins enter the bloodstream. One of the first steps in this process is the translocation of cecal bacteria to the mesenteric lymph nodes (MLN). As liquid feeding tends to increase the amount of gram-negative bacteria, it is important to maintain the blood-mucosal barrier to eliminate translocation. The effect to diet on this translocation was investigated, and results are presented below in Table 4B. Each group of mice was fed chow, Liq. Liq+S, or Liq+G as described above for 14 days.

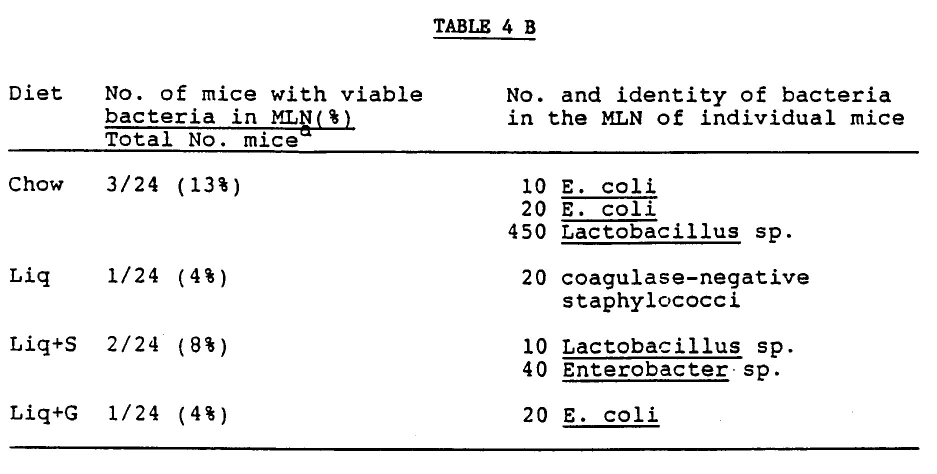

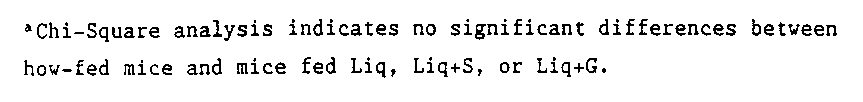

Thus, as shown in he table above, addition of the hydrolysed guar fiber did not have any adverse effects on the translocation of bacteria into the MNL.

### 3. Effect of Diet on Bacteria in Endotoxin Lipopolysaccharide-treated (LPS) mice

Each group of mice was fed as previously described and was given 200 »g i.p. injection of endotoxin lipopolysaccharide, the toxin which is involved in septic shock. Results are presented in Table 4C below. Abbreviations and units are the same as those used in Table 4A.

**TABLE 4C**

| Diet | Wt. | Aerobic + facultative gram-neg. bacilli | Aerobic + facultative gram-pos. bacteria | Strict Anaerobes |
|---|---|---|---|---|
| Chow | 0.3 | 9.0 ± 0.1 | 8.4 ± 0.2 | 9.7 ± 0.2 |
| Liq | 1.8 | 9.7 ± 0.3 | 9.4 ± 0.3^{a} | 10.2 ± 0.2 |
| Liq+S | 2.3 | 9.2 ± 0.2 | 8.7 ± 0.2 | 9.7 ± 0.2 |
| Liq+G | 1.2 | 9.1 ± 0.2 | 8.9 ± 0.2 | 10.2 ± 0.1 |

| | | | | |
|---|---|---|---|---|
| ^{a}Significantly increased compared to chow-fed mice P<0.1 by ANOVA | | | | |

None of the liquid diets were seen to have an adverse effect on the intestinal flora of LPS-treated mice. AS expected, number of enteric gram-negative bacteria (primarily E. coli) increased with the intraperitoneal LPS treatment for all treatment groups.

### 4. Translocation of bacteria to MLN in LPS-treted mice

Mice were fed as described supra and treated with LPS as described supra. The number and identity of bacteria found in the MLN was determined. Results are presented in Table 4D, below.

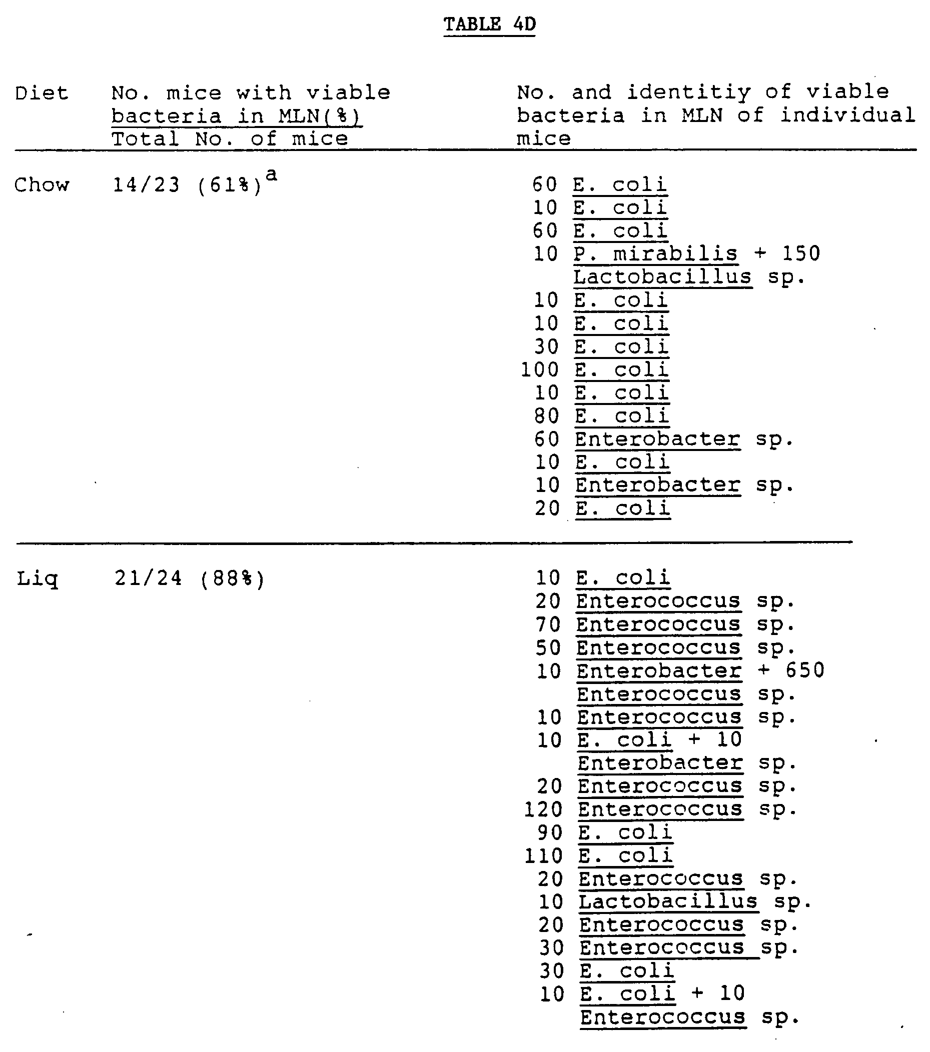

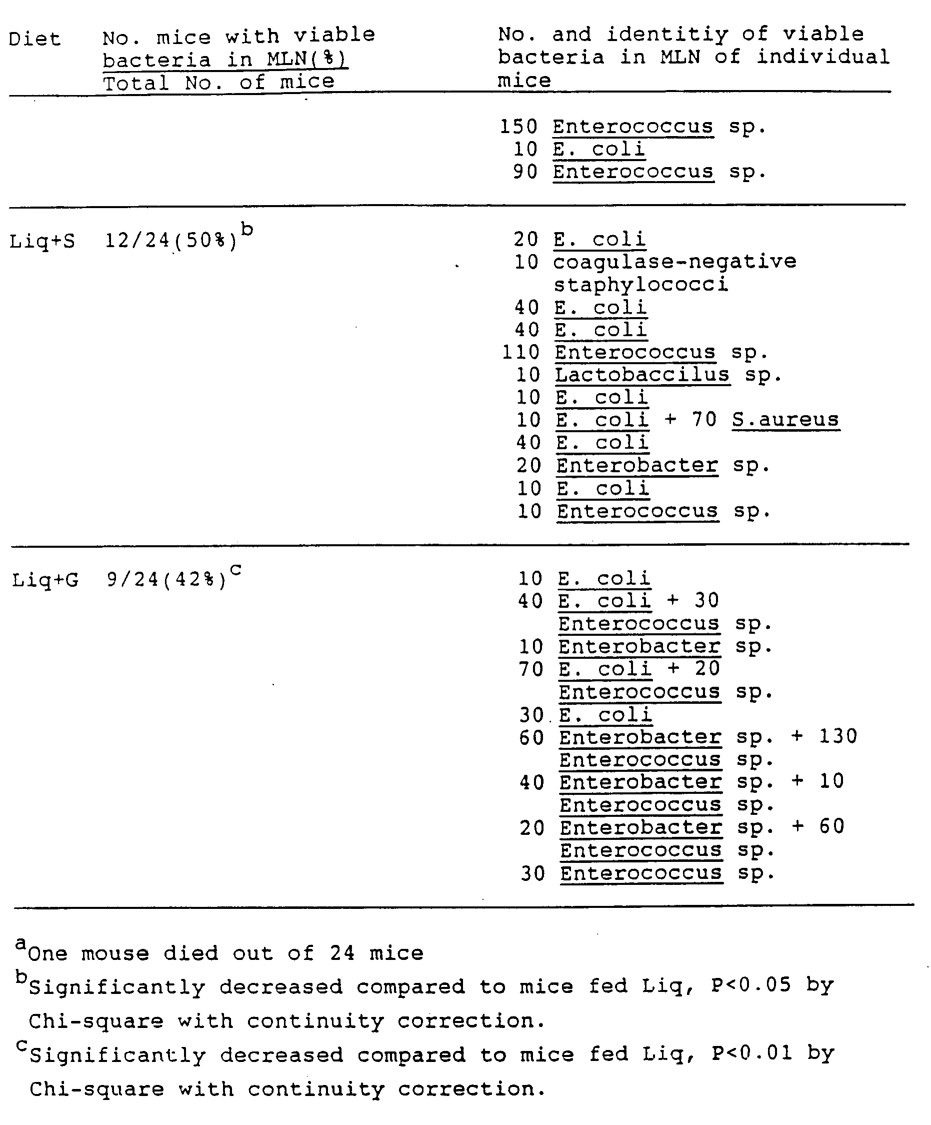

Compared to chow fed mice, Liq+S and Liq+G have an improvement in preventing the translocation of intestinal bacteria to the mesenteric lymph nodes of mice. Compared to chow-fed mice, Liq appeared to increase the incidence of bacterial translocation somewhat (P=0.08). However, the supplementation with soy or hydrolysed guar fiber had the beneficial effect of significantly decreasing the incidence of translocation of bacteria. The Liquid composition supplemented with the soy fiber is however not suitable for tube feeding.

### EXAMPLE 5

In a randomised, double blind, cross-over study the effect of supplementation of a tube-feeding formula with hydrolysed soluble fiber on gut transit time was investigated in 12 healthy volunteers.

The test diets were a self selected diet (SDS) the standard formula diet NUTRODRIP^{R} (SANDOZ Nutrition) and an identical formulation supplemented with 2 % by weight of SUNFIBER^{R} (21 g/l) The diets are administered, bolus-wise, in an iso-caloric amount covering the required energy supply (between 2000 and 2500 Kcal per day). The oral-coecum transit time (OCT) was assessed by lactulose H2 breath test, the colonic transit time (CTT) by a radioopaque marker technique. The stool frequency (SF) was recorded as well. The results shown in Table 5 indicate that hydrolysed soluble fibers are beneficial for enterally fed patients with diarrhea due to a prolongation of CTT without significantly affecting OCT. Daily SF was not different, but a trend towards increased SF was observed with NUTRODRIP^{R} whereas SUNFIBER^{R} reversed this effect.

The results are as follows:

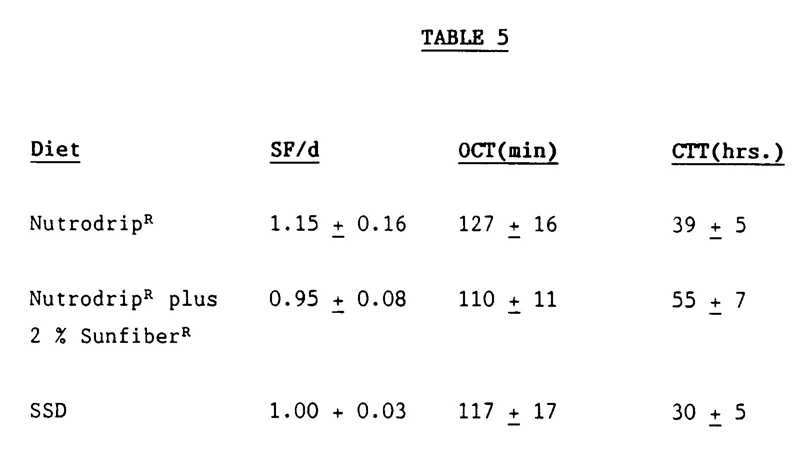

## Claims

1. A feeding composition which is nutritionally complete comprising hydrolysed soluble fiber which are able to undergo fermentation in the colon to produce short fatty acids in an amount such that the daily dosage of the feeding composition provides from 10 to 60 grams of hydrolysed soluble fiber per day.

2. The composition according to Claim 1 which is a liquid composition.

3. The composition according to Claim 2 which has a viscosity of less than 0,05 Pa·s (50 cp).

4. The composition according to Claim 3 which has a viscosity of less than 0,025 Pa·s (25 cp).

5. The composition according to Claims 1 to 4 providing from 10 to 45 grams of hydrolysed soluble fiber in a formulation volume providing up to 3000 kcal energy.

6. The composition according to Claim 5 providing from 10 to 45 grams of hydrolysed soluble fiber in a formulation volume providing from 1500 to 2500 kcal energy.

7. A composition according to Claims 1 to 6, wherein the soluble fiber is selected from the group consisting of hydrolysed guar gum and hydrolysed pectin.

8. A composition according to Claim 7 comprising hydrolysed guar gum.

9. An enteral composition according to Claims 1 to 8.

10. A low viscosity feeding composition according to Claims 1 to 9, comprising:
carbohydrates providing approximately 20-70 % of the total calories;
protein providing approximately 10-30 % of the total calories;
lipid containing essential fatty acids providing approximately 20-50 % of the total calories;
vitamins;
mineral;
water; and
hydrolysed soluble fiber.

11. The use of a hydrolysed soluble fiber which is able to undergo fermentation in the colon to produce short fatty acids for the manufacture of a nutritionally complete feeding composition for preventing bacterial sepsis or gut atrophy or for treating or preventing diarrhea in the human body.

## Patentansprüche

1. Nahrungszusammensetzung, die vollen Nährwert besitzt und eine hydrolysierte lösliche Faser umfaßt, die im Colon einer Fermentation unter Bildung von kurzen Fettsäuren unterzogen werden kann, in einer Menge, so daß die Tagesdosis der Nahrungszusammensetzung 10 bis 60 Gramm hydrolysierte lösliche Faser pro Tag bereitstellt.

2. Zusammensetzung nach Anspruch 1, die eine flüssige Zusammensetzung ist.

3. Zusammensetzung nach Anspruch 2, die eine Viskosität von weniger als 0,05 Pa·s (50 cp) aufweist.

4. Zusammensetzung nach Anspruch 3, die eine Viskosität von weniger als 0,025 Pa·s (25 cp) aufweist.

5. Zusammensetzung nach den Ansprüchen 1 bis 4, die 10 bis 45 Gramm hydrolysierte lösliche Faser in einem Formulierungsvolumen bereitstellt, das bis zu 3 000 kcal Energie liefert.

6. Zusammensetzung nach Anspruch 5, die 10 bis 45 Gramm hydrolysierte lösliche Faser in einem Formulierungsvolumen bereitstellt, das 1 500 bis 2 500 kcal Energie liefert.

7. Zusammensetzung nach den Ansprüchen 1 bis 6, worin die lösliche Faser aus der Gruppe ausgewählt wird, die aus hydrolysiertem Guaran und hydrolysiertem Pektin besteht.

8. Zusammensetzung nach Anspruch 7, die hydrolysiertes Guaran umfaßt.

9. Enterale Zusammensetzung nach Anspruch 1 bis 8.

10. Niedrigviskose Nahrungszusammensetzng nach den Ansprüchen 1 bis 9, die folgendes umfaßt:
Kohlenhydrate, die etwa 20-70 % der gesamten Kalorien bereitstellen,
Protein, das etwa 10-30 % der gesamten Kalorien bereitstellt,
Lipid, das essentielle Fettsäuren enthält und etwa 20-50 % der gesamten Kalorien bereitstellt,
Vitamine,
Mineralien,
Wasser und
hydrolysierte lösliche Faser.

11. Verwendung einer hydrolysierten löslichen Faser, die im Colon unter Bildung von kurzen Fettsäuren einer Fermentation unterzogen werden kann, zur Herstellung einer Nahrungszusammensetzung mit vollem Nährwert zur Verhinderung bakterieller Sepsis oder Darmatrophie oder zur Behandlung oder Verhinderung von Diarrhoe im menschlichen Körper.

## Revendications

1. Une composition nutritive qui est complète d'un point de vue nutritionnel, comprenant des fibres hydrolysées qui sont capables de subir une fermentation dans le côlon pour produire des acides gras courts, en une quantité telle que la dose quotidienne de la composition nutritive fournisse de 10 à 60 g de fibres solubles hydrolysées par jour.

2. La composition selon la revendication 1, qui est une composition liquide.

3. La composition selon la revendication 2, qui a une viscosité inférieure à 0,05 Pa.s (50 cp).

4. La composition selon la revendication 3, qui a une viscosité inférieure à 0,025 Pa.s (25 cp).

5. La composition selon les revendications 1 à 4, fournissant de 10 à 45 g de fibre soluble hydrolysée pour un volume de formulation fournissant jusqu'à 3000 kcal.

6. La composition selon la revendication 5, fournissant de 10 à 45 g de fibre soluble hydrolysée pour un volume de formulation fournissant de 1500 à 2500 kcal.

7. Une composition selon les revendications 1 à 6, dans laquelle la fibre soluble est choisie dans le groupe constitué par la gomme guar hydrolysée et la pectine hydrolysée.

8. Une composition selon la revendication 7, comprenant de la gomme guar hydrolysée.

9. Une composition entérale selon les revendications 1 à 8.

10. Une composition nutritive à faible viscosité selon les revendications 1 à 9, comprenant
- des glucides fournissant environ de 20 à 70% du total des calories,
- des protéines fournissant environ de 10 à 30% du total des calories,
- des lipides contenant des acides gras essentiels fournissant environ de 20 à 50% du total des calories,
- des vitamines,
- des sels minéraux,
- de l'eau, et
- une fibre soluble hydrolysée.

11. L'utilisation d'une fibre soluble hydrolysée qui est capable de subir une fermentation dans le côlon pour produire des acides gras courts, pour la préparation d'une composition nutritive complète d'un point de vue nutritionnel, pour empêcher la septicémie bactérienne ou l'atrophie des intestins ou pour le traitement ou la prévention de la diarrhée dans le corps humain.
